# EUROPÄISCHE PATENTSCHRIFT

(11) **EP 3 416 580 B1**
(45) Veröffentlichungstag und Bekanntmachung des Hinweises auf die Patenterteilung: **13.12.2023**
(21) Anmeldenummer: 17709591.6
(22) Anmeldetag: 17.02.2017
(51) Int. Cl.: A61B 18/20

(54) **VORRICHTUNG ZUR BESTRAHLUNG DER HAUT**
DEVICE FOR IRRADIATING THE SKIN
DISPOSITIF DE RAYONNEMENT POUR LA PEAU

(30) Priorität: 18.02.2016 EP 16156270
(43) Veröffentlichungstag der Anmeldung: 26.12.2018
(73) Patentinhaber: Fatemi, Afschin, 40545 Düsseldorf (DE)
(72) Erfinder: Fatemi, Afschin, 40545 Düsseldorf (DE)
(74) Vertreter: Roth, Andy Stefan
(86) Internationale Anmeldenummer: PCT/EP2017/053589
(87) Internationale Veröffentlichungsnummer: WO 2017/140829

(56) Entgegenhaltungen:
- EP-A1- 1 285 680
- EP-A4- 1 285 680
- WO-A1-2015/051999
- US-A- 5 786 924
- US-A1- 2003 036 680

## Beschreibung

Die Offenbarung betrifft eine Vorrichtung sowie ein Verfahren zur Bestrahlung einer Zielstruktur mit einer Laserstrahlung sowie eine geeignete Verwendung eines mit der Laserstrahlung erzeugten Brennflecks. Die Vorrichtung verfügt hierbei über wenigstens eine Laserlichtquelle und zumindest ein Lichtlenkmittel, die derart ausgeführt sind, dass Laserstrahlung über unterschiedliche Strahlengänge in die Zielstruktur gelangt, wobei aufgrund eines durch die Bestrahlung bedingten Energieeintrags zumindest bereichsweise eine Eigenschaftsveränderung der Zielstruktur erfolgt.

Die von einem Laser ausgesandten Strahlen zeichnen sich vor allem durch ihre hohe Intensität, den sehr engen Frequenzbereich der Strahlung und damit einhergehende targetspezifischen Selektivität, eine scharfe Bündelung des Strahls sowie die große Kohärenzlänge aus. Neben vielen bekannten Anwendungen im technischen Bereich, findet der Laser auch vielfach in der Medizintechnik Anwendung. So werden beispielsweise in der Dermatologie mit Hilfe von Laserstrahlen Schnitte und Verödungen durchgeführt. Ebenso können Blutgefäße durch Laser bestimmter Wellenlänge koaguliert werden und Pigmentflecken mit Hilfe ablatierender, bzw. sogenannter schälender Laser abgetragen oder selektiv zerstört werden. Ferner werden subkutane Pigmente mit Hilfe einer ultrakurzgepulsten Laserquelle zerstört und damit entfernt, ohne die Hautoberfläche nachhaltig zu verletzen, oder mit Hilfe von langgepulsten Lasern Haarwurzeln durch Epilation dauerhaft zerstört. Darüber hinaus werden Laser teilweise zur gezielten Behandlung entzündlicher Hauterkrankungen, wie etwa der Psoriasis (Schuppenflechte), eingesetzt oder es werden oberflächliche Unebenheiten der Haut, wie Knötchen oder Fältchen, zur kosmetischen Verbesserung des Hautbildes geglättet (Resurfacing). Gemäß einer weiteren Anwendung in der Dermatologie werden Laser verwendet, um selektiv dermale Anteile zu erwärmen und so den Kollagenaufbau zu fördern und die Haut in diesem Bereich zu straffen (Subsurfacing).

Je nach der Art des verwendeten Lasers kommt es zu unterschiedlichen Wechselwirkungen zwischen der Haut bzw. dem Gewebe und dem vom Laser ausgesendeten Licht. Die Art der Wechselwirkung hängt einerseits von den optischen Eigenschaften der Haut bzw. des Gewebes, insbesondere dem Streu- und dem Absorptionskoeffizienten sowie der Dichte, und andererseits von den physikalischen Parametern des Laserlichts, insbesondere der Wellenlänge, der Energiedichte, der Wiederholrate, der Bestrahlungsdauer sowie der Spotgröße, ab.

Die Laser-Gewebe-Wechselwirkungen werden in verschiedene Mechanismen eingeteilt. Hierzu gehören die photo-thermale, photo-mechanische bzw. photo-akustische und die photo-chemische Wechselwirkung. Weiterhin wird teilweise noch das Phänomen der photo-vermittelten Ablation, die sogenannte Photoablation, genutzt. Unter diesen Effekten ist die am meisten quantifizierbare und zumeist beobachtende Reaktion der photothermische Effekt, der durch die Einbringung hoher Energien und die dadurch verursachte Verdampfung von Wasser im Gewebe erreicht wird. Je nach Beschaffenheit des bestrahlten Mediums und der entsprechenden Struktur sowie der Bestrahlungsparameter treten die verschiedenen Effekte unterschiedlich stark auf, wobei angenommen wird, dass die absorbierte Energie und die Bestrahlungszeit die Gewebewirkung zum größten Teil beeinflussen. Die entsprechenden Wechselwirkungen werden im Detail in "C. Raulin, S. Kasei (Hrsg.), Lasertherapie der Haut, Springer-Verlag Berlin, Heidelberg 2013" beschrieben.

In diesem Zusammenhang wird in der US 2011/0313408 A1 ein Laser zur Behandlung der Haut beschrieben. Wesentlich an der beschriebenen technischen Lösung ist, dass für die Bestrahlung ein erster Laserstrahl mit langer Pulsdauer und ein zweiter Laserstrahl mit kurzer Pulsdauer unter gleichzeitigem Einsatz eines Kühlelements zum Kühlen eines Oberflächenbereichs der Haut verwendet werden. Mit dem ersten Laserstrahl wird ein Volumen von Hautgewebe unterhalb des gekühlten Oberflächenbereichs erwärmt, um das Hautgewebe noch unterhalb der Hautgewebeschädigungsschwelle zu modifizieren. Der zweite Laserstrahl wird in eine Vielzahl von getrennten Laserstrahlen mit Hilfe von getrennten optischen Fasern aufgeteilt und über getrennte Wege durch das Hautgewebe in das vorerwärmte Hautgewebe gelenkt, um hier in gezielt ausgewählten, kleinen Volumina mechanische Beschädigungen hervorzurufen.

Ferner ist aus der US 2007/0239147 A1 ein System zur Behandlung von dermatologischen Erkrankungen bekannt, das auf der thermischen Schädigung einer Zielstruktur beruht. Hierbei wird gezielt ein Strahlenbündel auf einen Zielpunkt auf der Hautoberfläche gerichtet, um die Zielstruktur im Gewebe unterhalb dieser Zielposition zu schädigen. Insbesondere sind Lichtlenkmittel vorgesehen, mit denen ein zweiter Lichtstrahl auf die gleiche Zielposition gelenkt wird, um ein anderes Gewebevolumen unterhalb des Zielortes thermisch zu schädigen.

Aus der US 7,066,929 B1 ist ferner eine selektive Photothermolyse bekannt, mit der subkutanes Gewebe durch Verwendung einer Mehrzahl von Strahlen von schmalbandigen, elektromagnetischen Wellen zerstört wird. Da jeder der Strahlen nicht über die ausreichende Energie verfügt, um das Gewebe auf die benötigte Temperatur zu erwärmen und durch Übererhitzung zu zerstören, werden die einzelnen Strahlen am Zielpunkt derart überlappt, so dass ausreichende Wärme erzeugt wird, um das Zielgewebe zu zerstören. Gemäß der beschriebenen technischen Lösung ist hierfür eine Strahllenkeinrichtung vorgesehen, die die einzelnen Lichtstrahlen derart lenkt, dass jeder der Strahlen beim Verlassen der Strahlungsvorrichtung in einem anderen Winkel austritt. Mit Hilfe einer geeigneten Steuerung des Lasers werden die unterschiedlichen einzelnen Strahlen schließlich in einem Punkt fokussiert.

Im Weiteren beschreibt die WO 2013/156421 A1 ebenfalls eine Vorrichtung zur Behandlung von Haut- und/oder Gewebeschichten mit Hilfe von Laserlicht. Wesentlich an der beschrieben Vorrichtung ist, dass eine Vielzahl von Laserlichtquellen verwendet wird, um gleichzeitig oder abwechselnd eine Zielstruktur aus unterschiedlichen Richtungen zu bestrahlen. Die Laserlichtquellen und/oder geeignete Lichtlenkmittel sind derart angeordnet, dass das Laserlicht auf unterschiedlichen Wegen in die zu behandelnde Haut- oder Gewebeschicht und im Bereich der Zielstruktur punktuell fokussiert wird.

Die US 2003/036680 A1 beschreibt einen Laser zur Behandlung der menschlichen Haut, wobei der Laserlichtstrahl ausgehend von einer Laserlichtquelle zunächst durch eine Öffnung in einem Konkav-Hohlspiegel auf einen Konvexpiegel und dann über den Kokav-Hohlspiegel in die Zielstruktur gelenkt wird. Gemäß der in der D1 beschriebenen technischen Lösung ist ferner ein Strahlteiler vorgesehen, sodass einerseits die vom Konkav-Hohlspiegel kommende Strahlung in Richtung der Zielstruktur umgelenkt wird und andererseits die von der Zielstruktur reflektierte Strahlung von einer CCD-Kamera aufgenommen werden kann.

Die WO 2015/051999 A1 beschreibt eine Vorrichtung zum Haarschneiden umfassend eine Hautberührungsseite, die eingerichtet ist, um gegen eine Oberfläche der Haut eines Benutzers während des Gebrauchs platziert zu sein, ein optisches System, das ausgelegt ist, um einen Schneidlaserstrahl über eine Schneidzone parallel zu und beabstandet von der die Haut berührenden Seite zu richten, um Haare zu schneiden, die sich in die Schneidzone erstrecken, einen Hautsensor, der konfiguriert ist, um eine oder mehr der optischen Eigenschaften der Oberfläche der Haut zu erfassen, und eine Steuereinheit, die konfiguriert ist, um ein oder mehr Merkmale des optischen Systems in Abhängigkeit von einer oder mehr der optischen Eigenschaften der Haut, die von dem Hautsensor erfasst werden, einzustellen.

Die EP 1 285 680 A1 beschreibt einen Laserstrahlprojektor zur Projektion eines Laserstrahls auf einen ausgewählten Bereich der Haut zur kosmetischen Behandlung.

Die US 5 786 924 A beschreibt eine Lasertherapiegerät zur Behandelung von Hautoberflächen.

Problematisch an den bekannten und derzeit zum Einsatz kommenden technischen Lösungen zur Behandlung der Haut ist oftmals, dass der Hautbereich, auf den die Laserstrahlung auftrifft, um von dort aus weiter in die Haut bzw. das subkutane Gewebe einzudringen, geschädigt wird. Derartige Schädigungen sind regelmäßig nicht erwünscht und können teilweise zu nicht unerheblichen Komplikationen führen. Weiterhin stellt es in vielen Fällen ein erhebliches Problem dar, gezielt größere, insbesondere subkutan in vergleichsweise großer Tiefe gelegene Zielstrukturen zu zerstören, ohne dass es zu Schädigungen von angrenzenden Gewebebereichen, Drüsen oder anderen Teilen der Haut oder des Gewebes kommt. Dies ist vor allem darauf zurückzuführen, dass es mit den bekannten technischen Lösungen nicht in ausreichendem Maße möglich ist, einen ausreichenden und trotzdem lokal begrenzten Energieeintrag in eine ausgedehnte Zielstruktur innerhalb der Haut oder des subkutanen Gewebes zu realisieren.

Ausgehend von dem aus dem Stand der Technik bekannten technischen Lösungen sowie den zuvor geschilderten Problemen liegt der Offenbarung die Aufgabe zu Grunde, eine Vorrichtung zur Behandlung der Haut eines Menschen oder eines Tieres mittels eines Lasers derart weiterzubilden, dass der benötigte Energieeintrag zumindest nahezu ausschließlich in die zu schädigende Zielstruktur erfolgt. Hierbei soll insbesondere eine Schädigung der Epidermis der Haut, auf die Laserstrahlung auftritt, vermieden werden und insgesamt der Energieeintrag in die Haut bzw. das Gewebe auf das jeweils benötigte Mindestmaß reduziert werden. Das beschriebene System soll sich weiterhin auf vergleichsweise einfache Weise in ein kompaktes Gerät integrieren lassen, und einen wirtschaftlichen Einsatz im täglichen Betrieb ermöglichen. Weiterhin soll das anzugebende System derart ausgeführt werden, dass ein flexibler Einsatz, insbesondere die Durchführung einer Vielzahl von unterschiedlichen Behandlungen der Haut und des darunter liegenden Gewebes möglich ist.

Die zuvor beschriebene Aufgabe wird mit einer Vorrichtung gemäß Anspruch 1 gelöst.

Ein geeignetes Verfahren zur Erzeugung eines Brennpunktes oder eines Brennflecks in einer Zielstruktur ist im Anspruch 12 sowie eine auf der Erfindung beruhende Verwendung eines entsprechend erzeugten Laserstrahls ist in Anspruch 13 angegeben. Vorteilhafte Ausführungsformen der Erfindung sind in den abhängigen Ansprüchen angegeben und werden in der folgenden Beschreibung unter teilweiser Bezugnahme auf die Figuren näher erläutert.

Die nachstehenden technischen Informationen können in mancher Hinsicht über den Umfang der Erfindung hinausgehen, der ausschließlich durch die beigefügten Ansprüche definiert ist. Die zusätzlichen technischen Informationen dienen dazu, die eigentliche Erfindung in einen breiteren technischen Kontext zu stellen und mögliche verwandte technische Entwicklungen zu veranschaulichen. Alle zusätzlichen technischen Informationen, die nicht in den Anwendungsbereich der beigefügten Ansprüche fallen, werden als solche nicht beansprucht und sind nicht als Teil der Erfindung zu verstehen.

Die Erfindung betrifft eine Vorrichtung zur Bestrahlung einer Zielstruktur, die sich innerhalb von menschlicher oder tierischer Haut oder Gewebe, insbesondere subkutanem Gewebe, befindet. Die Vorrichtung verfügt über wenigstens eine Laserlichtquelle und zumindest ein Optikelement, mittels dem die Laserstrahlung durch eine Lichtaustrittsöffnung geleitet und in einem Brennpunkt außerhalb der Lichtaustrittsöffnung fokussiert wird. Weiterhin ist wenigstens ein Stellelement vorgesehen, mit dem der Abstand zwischen einer Hautoberfläche und dem im Bereich der Zielstruktur positionierten Brennpunkt einstellbar ist. Erfindungsgemäß ist die Vorrichtung derart weitergebildet worden, dass das Optikelement eine Anordnung eines konvexen Spiegels innerhalb eines Hohlspiegels aufweist, die derart angeordnet sind, dass von der Laserlichtquelle emittierte Laserstrahlung zumindest teilweise durch eine Öffnung im Hohlspiegel hindurchtritt, die durch die Öffnung hindurchgetretene Laserstrahlung auf den konvexen Spiegel trifft, vom konvexen Siegel zum Hohlspiegel gelenkt wird und vom Hohlspiegel kommend die Austrittsöffnung durchquert.

Die erfindungsgemäße Anordnung eines konvexes Spiegels innerhalb eines Hohlspiegels entspricht im Wesentlichen der Ausgestaltung eines Schwarzschild-Objektives, wobei allerdings der Strahlengang des Laserstrahls von der Laserlichtquelle bis zur Austrittsöffnung und weiter zum Brennpunkt des Hohlspiegels verläuft. Das erfindungsgemäß verwendete Optikelement verfügt somit über zwei einander gegenüber liegende, zentrisch angeordnete Spiegel bzw. Reflektorelemente. Der Hauptspiegel ist als konkaver Hohlspiegel konzipiert, dessen Spiegelfläche in Richtung der Lichtaustrittsöffnung ausgerichtet ist. Der deutlich kleinere, konvex geformte Spiegel, auch Auffangspiegel bzw. Auffangreflektor genannt, liegt als Wölbspiegel in einer Position nahe der Lichtaustrittsöffnung, wobei seine Spiegelfläche zum Hauptspiegel weist.

Das von der Laserlichtquelle ausgesandte Laserlicht fällt somit durch eine Öffnung im Hohlspiegel auf den kleinen Auffangspiegel, wird von diesem in radialer bzw. kugel- oder halbkugelförmiger Richtung auf den Hohlspiegel umgelenkt, der die Laserstrahlung schließlich durch die Austrittsöffnung in die Umgebung der Vorrichtung leitet.

In diesem Zusammenhang ist es denkbar, dass der Hohlspiegel entweder als Parabolspiegel oder als sphärischer Hohlspiegel ausgeführt ist. Hierbei zeichnet sich ein Parabolspiegel dadurch aus, dass alle Lichtstrahlen, die parallel zur optischen Achse auf den Spiegel auftreffen exakt im Brennpunkt gebündelt werden. Ein sphärischer Hohlspiegel, der die Form eines Kugelausschnitts aufweist, ist dagegen vergleichsweise einfach und damit kostengünstig herzustellen. Grundsätzlich ist es von Vorteil, den Hohlspiegel einstückig auszuführen. Generell ist aber auch eine mehrteilige, insbesondere zweiteilige Ausgestaltung denkbar, wobei in jedem Fall vorzugsweise mittig eine Öffnung vorzusehen ist, durch die die von der Laserlichtquelle kommende Strahlung hindurchtreten kann, um auf den konvexen Spiegel auftreffen zu können.

Der Mittelpunkt des konvexen Spiegels, insbesondere die der Laserlichtquelle am nächsten liegende Stelle, befindet sich bevorzugt auf der optischen Achse des von der Laserlichtquelle emittierten Laserstrahls.

Um den Brennpunkt innerhalb der Zielstruktur zu positionieren, muss die Laserlichtquelle mit dem Optikelement in einem entsprechenden Abstand zur Zielstruktur überführt werden. Oftmals befindet sich die Zielstruktur nicht direkt auf der Hautoberfläche, sondern liegt unterhalb der Hautoberfläche, etwa im subkutanen Gewebe. Entsprechend der Lage der Zielstruktur muss die Eindringtiefe der für die Behandlung vorgesehenen Laserstrahlung und hier insbesondere der Abstand des Brennpunktes von der Hautoberfläche eingestellt werden. Unter Eindringtiefe der Strahlung wird in diesem Zusammenhang regelmäßig die Tiefe des Brennpunkts innerhalb des Körpers des Patienten, also der Abstand des Brennpunktes von der Hautoberfläche verstanden.

Sofern sich die Zielstruktur nicht unmittelbar auf der Hautoberfläche befindet, hat dies in Bezug auf die auf die Hautoberfläche auftreffende Laserstrahlung den Effekt, dass die Auftrittsfläche auf der Hautoberfläche eine Kreisring- oder Ellipsenform aufweist, während die Strahlung im Brennpunkt, der sich dann unterhalb der Hautoberfläche in der Zielstruktur befindet, fokussiert wird. Der Energieeintrag auf die Hautoberfläche, die nicht behandelt werden soll und für die eine nachhaltige Schädigung verhindert werden soll, wird somit minimiert. Dagegen wird der maximale Energieeintrag innerhalb der Zielstruktur, nämlich im Brennpunkt erreicht. In diesem Zusammenhang wird darauf hingewiesen, dass die Strahlung gemäß der Erfindung regelmäßig nicht exakt in einem Brennpunkt sondern vielmehr in einem Brennbereich oder Brennvolumen fokussiert wird, wobei in diesem Bereich die Intensität der eingestrahlten Laserstrahlung maximal ist. Wesentlich für die Positionierung des Brennpunktes bzw. des Brennbereiches in der Zielstruktur ist somit das erfindungsgemäß vorgesehene Stellelement, mit dem der Abstand zwischen einer Hautoberfläche und dem im Bereich der Zielstruktur positionierten Brennpunkt gezielt einstellbar ist. In der einfachsten Ausgestaltung wird diese Einstellung dadurch erreicht, dass die Laserlichtquelle mit dem Optikelement und der Austrittsöffnung, die bevorzugt in und/oder an einem Gerätegehäuse angeordnet sind, manuell oder durch einen Roboterarm in eine Position überführt wird, sodass der Brennpunkt bzw. der Brennbereich innerhalb der Zielstruktur zu liegen kommt Um den benötigten Abstand zur Hautoberfläche einstellen zu können ist ein Stellelement vorgesehen, mit dem der Abstand zwischen einer Hautoberfläche und dem im Bereich der Zielstruktur positionierten Brennpunkt gezielt einstellbar ist.

In einer speziellen Weiterbildung der Offenbarung ist das Stellelement als Abstandhalter ausgebildet, der den jeweils benötigten Abstand zwischen der Hautoberfläche und der Lichtaustrittsöffnung einstellt. Hierfür ist wesentlich, dass durch eine Veränderung des Abstandes zwischen der Lichtaustrittsöffnung und der Hautoberfläche, zumindest bei unveränderten Optikeinstellungen, gleichzeitig der Abstand zwischen der Hautoberfläche und dem Brennpunkt verändert wird. Auf diese Weise kann durch Wahl des geeigneten Abstandhalters die Eindringtiefe des Brennpunktes der Strahlung innerhalb der Haut bzw. dem unterhalb der Haut gelegenen Gewebe verändert werden. Grundsätzlich ist es in diesem Zusammenhang denkbar, eine Mehrzahl von Abstandhaltern unterschiedlicher Größe, insbesondere unterschiedlicher Länge vorzusehen, die beispielsweise an einem Gehäuse der erfindungsgemäß ausgeführten Vorrichtung austauschbar befestigt werden.

In einer weiteren Ausführungsform der Offenbarung ist vorgesehen, dass der Abstandhalter einen Aktuator aufweist, über den der Abstandhalter wenigstens teilweise derart bewegbar ist, dass wenigstens zwei unterschiedliche Abstände zwischen der Austrittsöffnung und der Haut einstellbar sind. Ein derartiger Aktuator kann beispielsweise in Form eines Hebels oder Stellrades mit daran gekoppelten bewegbaren Elementen ausgeführt sein, sodass eine Länge des Abstandhalters auf den gewünschten Abstand zwischen Lichtaustrittsöffnung und Hautoberfläche einstellbar ist. Alternativ oder in Ergänzung ist es denkbar, dass der Aktuator bevorzugt elektrisch angetriebene Elemente, beispielsweise einen Elektromotor, der bei Bedarf mit einem Getriebe kombiniert werden kann, aufweist, über die der Abstandhalter auf die entsprechende Länge ausgefahren werden kann, bzw. mit denen der benötigte Abstand zwischen der Lichtaustrittsöffnung und der Hautoberfläche eingestellt werden kann.

Eine weitere Ausführungsform der Offenbarung sieht vor, dass das Stellelement wenigstens ein Bewegungsmittel aufweist, mit dem wenigstens ein Bauteil des Optikelementes derart bewegbar ist, dass der Abstand zwischen der Austrittsöffnung und dem Brennpunkt aufgrund der Bewegung verstellt wird. Diese Ausführungsform bietet den Vorteil, dass die Vorrichtung in gleichbleibendem Abstand auf die Hautoberfläche aufgesetzt werden kann und die Eindringtiefe des Brennpunktes innerhalb der Haut durch eine Verstellung wenigstens eines Bauteils des Optikelementes verstellbar ist. Bevorzugt ist das Bewegungsmittel mit wenigstens einem der beiden Spiegel des Optikelementes mechanisch verbunden und kann wenigstens einen dieser Spiegel derart bewegen, dass die Eindringtiefe der Laserstrahlung auf den benötigten Wert eingestellt werden kann. Selbstverständlich ist auch der Einsatz pneumatisch oder elektromagnetisch angetriebener Bewegungsmittel denkbar.

In einer besonderen Ausführungsform ist ferner zumindest ein Kühlelement vorgesehen, mit dem eine Hautoberfläche, insbesondere eine Oberfläche der Hornschicht, kühlbar ist. Der Einsatz eines derartigen Kühlelementes, mit dem die Hautoberfläche, auf die die Laserstrahlung auftrifft, gezielt gekühlt wird, bietet den Vorteil, dass einer Erwärmung der Haut durch die auftreffende Laserstrahlung entgegen gewirkt werden kann. In diesem Zusammenhang ist es auf vorteilhafte Weise denkbar, dass die Kühlung der Haut geregelt erfolgt, wobei für die Regelung der Kühlung die jeweilige Leistung des von der Laserlichtquelle ausgesandten Laserlichts berücksichtigt wird.

Weiterhin bevorzugt ist eine Steuereinheit vorgesehen, die zumindest zeitweise ein Steuersignal an die Laserlichtquelle und/oder das Stellelement überträgt. Mit einer derartigen Steuereinheit ist es möglich, einerseits die Intensität der von der Laserlichtquelle ausgesandten Laserstrahlung und andererseits die Eindringtiefe der Laserstrahlung in die Haut bzw. in das subkutane Gewebe gezielt zu verändern und so an die patientenspezifischen Bedürfnisse bzw. die geplante Behandlung anzupassen.

Ferner ist vorzugsweise ein Datenspeicher vorgesehen, der sich innerhalb der Steuereinheit befindet oder zumindest mit dieser verbunden ist, und in dem Eigenschaften des zu behandelnden bzw. des zu be- oder durchstrahlenden Gewebes abgelegt sind. Insbesondere sind hier optische Eigenschaften verschiedener Haut- und/oder Gewebebereiche oder entsprechender Haut- und/oder Gewebetypen abgelegt, sodass die Laserlichtquelle und/oder das Optikelement mit Hilfe eines von der Steuereinheit erzeugten Steuersignals derart eingestellt werden können, dass eine gezielte Behandlung der Zielstruktur erfolgt, ohne das umliegende, nicht zu behandelnde Gewebe nachhaltig geschädigt werden.

Bevorzugt werden Werte für spezielle optische Eigenschaften, wie beispielsweise der Brechungsindex und/oder das Absorptionsvermögen verschiedenartiger Haut- oder Gewebetypen in dem Datenspeicher abgelegt. Auf vorteilhafte Weise können die benötigten Werte vor Behandlungsbeginn vom Anwender ausgewählt und automatisch der Ansteuerung der Laserlichtquelle und/oder des Optikelements zu Grunde gelegt werden.

In einer speziellen Weiterbildung der Offenbarung handelt es sich bei der Laserlichtquelle um eine Lichtquelle, die hinsichtlich der Intensität und/oder der emittierten Lichtwellenlänge geregelt werden kann. Bevorzugt weist die Laserlichtquelle einen He-Ne-Laser, einen Nd-YAG-Laser und/oder einen Er-YAG-Laser.

Gemäß einer besonderen Ausgestaltung der Offenbarung ist vorgesehen, dass wenigstens zwei, vorzugsweise eine noch größere Mehrzahl von Laserquellen verwendet wird, um einerseits den zu behandelnden Bereich zu vergrößern und/oder andererseits unterschiedliche potentielle Wegstrecken vorzusehen, über die die Laserlichtstrahlung durch die Haut und den Körper des Patienten in den zu behandelnden Bereich gelangt. In diesem Zusammenhang ist es vorteilhaft, wenn den entsprechenden Laserlichtquellen ein erfindungsgemäß ausgeführtes Optikelement mit den beiden zuvor beschriebenen Spiegeln zugeordnet ist. Weiterhin ist es von Vorteil, wenn zumindest eine der Laserlichtquellen und/oder das jeweils zugeordnete Optikelement bewegbar ausgeführt sind. Die Bewegung erfolgt beispielsweise mit einem geeigneten Stellmotor, der durch die Steuereinheit auf der Art und des Fortschritts der Behandlung angesteuert wird. In einer ganz besonderen Weiterbildung ist vorgesehen, eine Mehrzahl von Laserlichtquellen mit daran befestigten Optikelementen vorzusehen, auf einem Kreis oder Kreisabschnitt angeordnet und an einem entsprechenden Gestell befestigt sind. Hierbei können wahlweise das Gestell und/oder die daran befestigten Baugruppe, zumindest bestehend aus Laserlichtquelle und Optikelement, bewegbar ausgeführt sein.

Vorzugsweise befinden sich die zuvor beschriebenen Bauteile einer offenbarungsgemäßen ausgeführten Vorrichtung in einem Gerätegehäuse, das zumindest einen Handgriff zur manuellen Führung und Betätigung der Vorrichtung aufweist. Vorzugsweise ist im Bereich des Griffes wenigstens ein Schaltelement vorgesehen, durch das die Laserlichtquelle eingeschaltet und/oder gewünschte Parameter, insbesondere als Führungs- oder Sollwerte eingegeben werden können. Gemäß einer speziellen Weiterbildung der Offenbarung können durch dieses Schaltelement oder ein weiteres Schaltelement auch gezielt Einstellungen des Stellelements und/oder des Optikelements verändert werden, so dass ein Abstand zwischen der Lichtaustrittsöffnung und dem Brennpunkt auf den benötigten Wert einstellbar ist.

Weiterhin bevorzugt verfügt das Gerätegehäuse über wenigstens eine Anzeigeeinheit, insbesondere ein Display, zur Anzeige von im Datenspeicher abgelegten, auswählbaren Parametern, während der Verwendung der Vorrichtung Istwerten auftretenden und/oder voreingestellten oder eingegebenen Sollwerten.

Neben einer Vorrichtung betrifft die Erfindung auch ein Verfahren zur Erzeugung eines Brennflecks oder Brennpunkts zur gezielten Veränderung wenigstens einer Materialeigenschaft eines Bauteils in einer Zielstruktur. Bei Verwirklichung des erfindungsgemäßen ergibt sich die im Folgenden angegebene Reigenfolge von Verfahrensschritten:
- Erzeugen einer Laserstrahlung mit einer Laserlichtlichtquelle und Lenkung der Laserstrahlung mit Hilfe eines Optikelement zu einer Lichtaustrittsöffnung, sodass die Strahlung außerhalb der Lichtaustrittsöffnung in dem Brennpunkt oder Brennfleck fokussiert wird und
- Einstellen eines Abstands zwischen einer Bauteiloberfläche und dem im Bereich der Zielstruktur positionierten Brennfleck oder Brennpunkt mittels eines Stellelements.

Das offenbarungsgemäße Verfahren zeichnet sich dadurch aus, dass die von der Laserlichtquelle emittierte Laserstrahlung zumindest teilweise von einer Rückseite kommend, die einer Spiegelfläche gegenüber liegt, durch eine Öffnung in einem Hohlspiegel hindurchtritt, die durch die Öffnung hindurchgetretene Laserstrahlung einen konvexen Spiegel gelenkt wird, vom konvexen Siegel auf die Spiegelfläche des Hohlspiegels geleitet wird und vom Hohlspiegel kommend durch die Austrittsöffnung zum Brennfleck gelenkt wird.

Mit Hilfe der zuvor beschriebenen Vorrichtung sowie des Verfahrens können auf bevorzugte Weise Eigenschaftsveränderungen eines Materials in einer Zielstruktur, insbesondere in einer Zielstruktur, die in der Haut und/oder in subkutanem Gewebe eines Menschen oder eines Tieres liegt, verändert werden. In diesem Zusammenhang lässt sich die offenbarungsgemäße Vorrichtung und insbesondere die von dieser Vorrichtung im Bereich des Brennpunktes oder Brennflecks erzeugte Laserstrahlung zur Behandlung von Akne, Hautverunreinigungen, Hämangiomen, Zellulitis, Hyperhidrose, Hautkrebs, Hautfalten, Varicosis, Bandscheibenprolaps und/oder Fett verwenden.

Im Folgenden wird die Offenbarung ohne Beschränkung des allgemeinen Erfindungsgedankens anhand eines Ausführungsbeispiels unter Bezugnahme auf die Figuren näher erläutert.

Dabei zeigen:
- Figur 1:: Handführbares Gerät mit einer offenbarungsgemäß ausgeführten Vorrichtung zur Erzeugung eines in eine Zielstruktur gerichteten Laserstrahls;
- Figur 2:: schematische Darstellung einer Laserlichtquelle mit einem Optikelement, das eine offenbarungsgemäße Strahlführung ermöglicht sowie
- Figur 3:: Anordnung zur Durchführung einer dermatologischen Behandlung mit einer Mehrzahl von eine Laserlichtquelle und ein Optikelement aufweisenden Baugruppen.

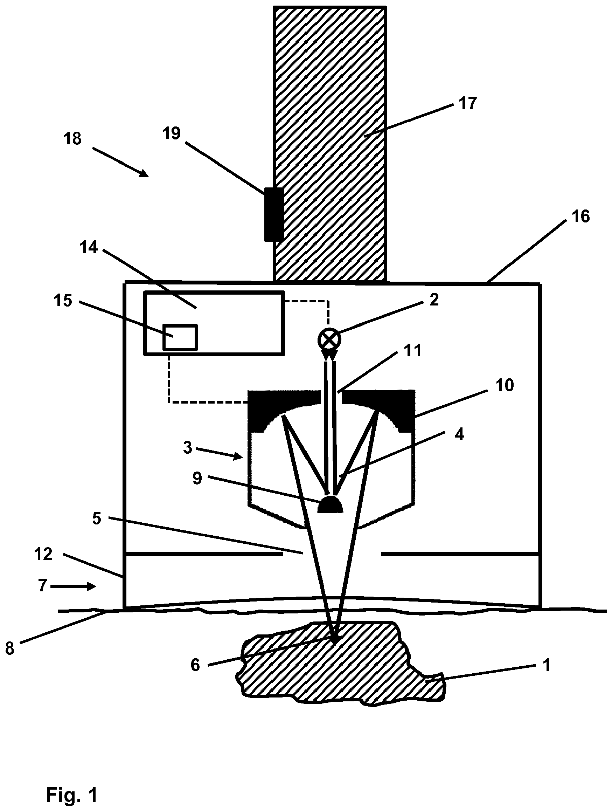

Figur 1 zeigt eine erfindungsgemäße Vorrichtung, die als handführbares Gerät 18 zur Durchführung von dermatologischen Behandlungen mittels Laserlicht 4 ausgebildet ist. Das Bestrahlungsgerät 18 kann mit der Hand bedient und geführt werden und wird zur Behandlung über den zu bestrahlenden Bereich mit der Zielstruktur 1 gehalten. Das dargestellte Bestrahlungsgerät 18 verfügt im Wesentlichen über einen Gehäusekörper 16, in dem eine Laserlichtquelle 2, ein Optikelement 3 und eine Lichtaustrittsöffnung 5 angeordnet sind, sowie über ein Stellelement 7 ein Handgriff 17 zur Führung des Geräts, die am Gehäuse 16 befestigt sind. Im Bereich des Handgriffs 17 ist als Schaltelement 19 ein Handschalter vorgesehen, über das die Laserlichtquelle 2 ein und ausgeschaltet werden kann.

Mit dem dargestellten Handbestrahlungsgerät 18 ist es möglich, auf einfache Weise eine Behandlung von kranker Haut oder subkutanem Gewebe durchzuführen. Innerhalb des Gerätegehäuses 16 ist eine Laserlichtquelle 2 zur Erzeugung von Laserstrahlung 4 vorgesehen. Der Laserstrahl 4 wir durch eine Öffnung 11 in einem Hohlspiegel 10 von dessen Rückseite kommend auf einen konvexen Spiegel 9 geleitet, der im Inneren des Hohlspiegels 10 auf der optischen Achse der Laserstrahlung 4 angeordnet ist. Von diesem konvexen Spiegel 9 wird die Laserstrahlung 4 auf die Spiegelfläche des Hohlspiegels 10 und von dort durch die in der Wand des Gehäuses 16 vorgesehenen Lichtaustrittsöffnung 5 gelenkt, so dass die Strahlung 4 im Bereich des Brennpunktes oder Brennflecks 6 fokussiert wird.

Als Stellelement 7 ist ein austauschbar am Gehäuse befestigter Abstandhalter 12 vorgesehen, der auf die Hautoberfläche 8 des Patienten oberhalb der Zielstruktur 1 aufgesetzt wird und einen festen Abstand zwischen der Lichtaustrittsöffnung 5 und der Hautoberfläche 8 festlegt, sodass auch der Abstand zwischen Hautoberfläche 8 und Brennpunkt oder Brennfleck 6 einen bestimmten, dem Nutzer bekannten Abstand aufweist. Hierbei sind auf vorteilhafte Weise unterschiedliche Abstandhalter 18 vorhanden, die in Abhängigkeit des Bedarfs, insbesondere in Abhängigkeit der benötigten Eindringtiefe in die Haut oder in das darunter befindliche Gewebe, wechselweise am Gehäuse 16 des Bestrahlungsgeräts 18 befestigt werden können und jeweils einen vorgegebenen Abstand zwischen der Lichtaustrittsöffnung 5 und der Hautoberfläche 8 einstellen. Ebenso ist es denkbar, ein Stellelement 7 vorzusehen, das am Gehäuse 16 des Bestrahlungsgeräts 18 befestigt ist und das über bewegbare Bauteile verfügt, so dass der Abstand zwischen der Lichtaustrittsöffnung 5 und der Hautoberfläche 8 bedarfsgerecht und gezielt eingestellt werden kann. Eine Verstellung kann manuell, bspw. mit Hilfe eines Schraubmechanismus, oder mit Hilfe eines Antriebselements, wie etwa einem Elektromotor, erfolgen.

Der maximale Außendurchmesser des Handbestrahlungsgeräts 18 im Bereich des Gehäusekörpers 16 beträgt etwa 6 cm. Bei einer besonderen konstruktiven Gestaltung liegt dieser Durchmesser bei etwa 3,5 bis 5 cm.

In der Steuerung 14, in der Steuersignale zum Betrieb der Laserlichtquelle 2 erzeugt werden, sind in einem Datenspeicher 15 optische Eigenschaften des zu be- und durchstrahlenden Gewebes und der Haut hinterlegt. Vor Behandlungsbeginn wird vom Anwender ein Behandlungsbereich innerhalb der Haut oder des subkutanen Gewebes lokalisiert und eine geeignete dreidimensionaler Zielstruktur 1 festgelegt, die bestrahlt werden soll. Im Weiteren werden für die Bestrahlung die festgelegten Strahlungsparameter der Laserstrahlung 4 sowie die in der Steuerung 14 hinterlegten optischen Eigenschaften der durchstrahlten Bereiche, unter anderem der Hautoberfläche 8, einerseits und der Zielstruktur 8 andererseits berücksichtigt. Auf diese Weise wird sichergestellt, dass während der Behandlung in der Haut bzw. im subkutanen Gewebe zumindest ein räumlicher Brennfleck 6 erzeugt wird, in dem die gewünschten Eigenschaftsveränderungen bewirkt werden.

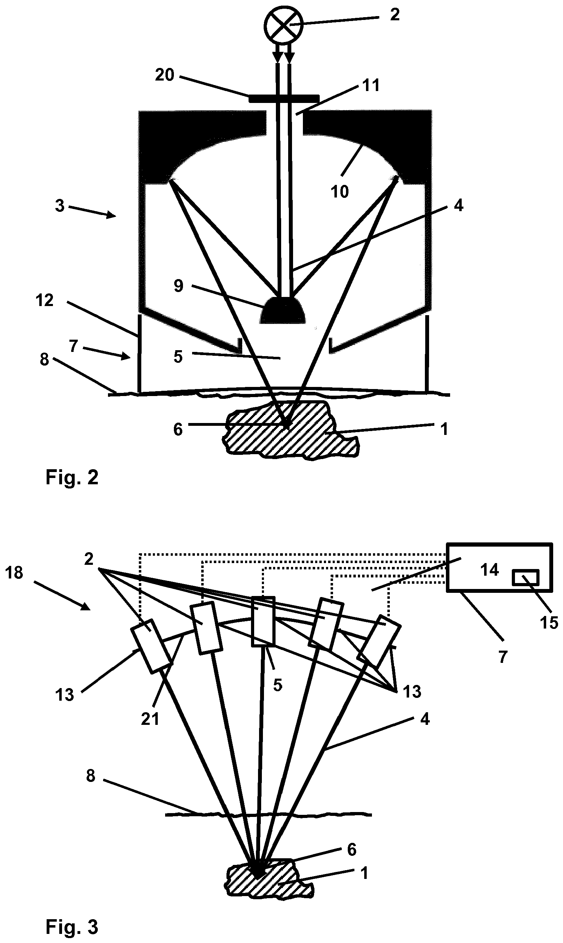

In Figur 2 wird ist eine schematische Darstellung einer Laserlichtquelle 2 mit einem Optikelement 3, das eine offenbarungsgemäße Strahlführung ermöglicht, gezeigt. Mit Hilfe der dargestellten Vorrichtung wird innerhalb der Zielstruktur 1 ein Brennpunkt oder ein Brennfleck 6 mit räumlicher Ausdehnung erzeugt. Wesentlich ist in diesem Fall, dass die von der wenigstens einen Laserlichtquelle 2 ausgesandte Strahlung 4 mit Hilfe eines Hohlspiegels 10 und einem mittig in dem Hohlspiegel angeordneten Konvexspiegel 9, die wie bei einem Schwarzschild-Objektiv angeordnet sind, auf den zu behandelnden Bereich, insbesondere in die Zielstruktur 1 gelenkt wird.

Die in diesem Fall für das Optikelemente verwendete Anordnung aus Hohlspiegel 10 und Konvexspiegel 9 zeichnet sich vor allem dadurch aus, dass ein Lichtstrahl 4, der eine inhomogene Intensitätsverteilung mit höherer Intensität in der Strahlmitte als am Strahlrand aufweist, wie es beispielsweise bei Lichtstrahlen, die von Excimerlasern emittiert werden, der Fall ist, verkleinert abgebildet wird. Excimerlaser emittieren kohärente UV-Lichtstrahlen hoher Intensität und werden daher vielfach in der Medizin eingesetzt.

Von großer Bedeutung für die offenbarungsgemäße Anordnung der Spiegel 9, 10 des Optikelements 3 sind die beiden einander gegenüberliegend, zentrisch angeordneten Spiegel 9, 10. Der Hauptspiegel ist als Hohlspiegel 10 konzipiert, dessen Spiegelfläche zur Lichtaustrittsöffnung 5 bzw. zur Hautoberfläche 8 gerichtet ist. Der deutlich kleinere Auffangspiegel 9 liegt als Wölb- bzw. Konvexspiegel in Richtung zur Lichtaustrittsöffnung 5, wobei seine Spiegelfläche zum Hauptspiegel 10 zeigt.

Die von der Laserlichtquelle 2 emittierten Lichtstrahlen 4 gelangen zunächst in eine Diffusoroptik 20, durch die eine Intensitätsverteilung des Lichtstrahls 4 vor Eintritt in die Anordnung aus Hohl- 10 und Konvexspiegel 9 derart verändert wird, dass der Lichtstrahl 4 in der Mitte eine geringere Intensität als in den Randbereichen aufweist. Die Diffusoroptik 20 kann hierfür etwa über ein Biprisma und einen Homogenisierer verfügen. Anschließend werden die Lichtstrahlen 4 mit Hilfe des konvexen Auffangspiegels 9 und des Hohlspiegels 10 auf die festgelegte Zielstruktur 1 fokussiert.

Wesentlich für die gezeigte technische Lösung ist, dass die Laserstrahlung 4 im Bereich der Zielstruktur 1 fokussiert wird, sodass die Strahlungsintensität im Bereich eines in der Zielstruktur 1 liegenden Brennflecks 6 ein Maximum annimmt, und dass dagegen diejenigen Haut- und Gewebebereiche, die nicht nachhaltig verändert werden sollen, nur einer vergleichsweise geringen Strahlungsleistung bzw. Strahlendosis ausgesetzt werden. Dies ist auf die besondere Strahlführung zurückzuführen, bei der die Strahlung 4 insbesondere im Bereich der Hautoberfläche 8 auf eine vergleichsweise große Kreisringfläche verteilt wird. Eine Fokussierung der Laserstrahlung 4 kann somit zielgenau in der Zielstruktur 1 erfolgen.

Um in speziellen Fällen eine zusätzliche Maßnahme zum Schutz der Haut, auf die zumindest zeitweise ein Teil der Laserstrahlung auftrifft, vorzusehen, ist in Abhängigkeit der gewählten Behandlung ein Kühlelement vorgesehen, das die Hautoberfläche 8 kühlt und so zusätzlich vor einer ungewünschten Schädigung durch die Laserbehandlung schützt.

Figur 3 zeigt eine spezielle Anordnung von Baugruppen, die jeweils eine Laserlichtquelle 2 und ein Optikelement 3 mit zwei Spiegeln, die, wie von einem Schwarzschild-Objektiv bekannt, angeordnet sind, aufweisen. Die Laserlichtquellen 2 mit den diesen nachgeschalteten Optikelementen 3 sind an einem Geräteträger 21 befestigt, wobei jeweils Bewegungsmittel 13 mit Stellmotoren vorgesehen sind, die auf der Grundlage eines von einer zentralen Steuereinheit 14 erzeugten Steuersignals die Laserlichtquellen 2 mit den Optikelementen 3 bedarfsgerecht bewegen. Mit Hilfe einer derartigen Anordnung mehrerer Lichtquellen 2, die gemäß dem in Figur 3 gezeigten Ausführungsbeispiel angeordnet sind, ist es einerseits möglich in einer Behandlungsphase eine besonders großflächige Bestrahlung durchzuführen, anderseits wird es so ermöglicht, Laserlichtstrahlen 4 auf unterschiedlichen Strahlungswegen in den Körper einzustrahlen. Durch diese Maßnahme kann wahlweise die Intensität einer Bestrahlung während einer Behandlung über einen großen Bereich variiert werden, es können zeitgleich unterschiedliche Bereiche einer Zielstruktur bestrahlt werden und/oder die Strahlungsbelastung der oberen Hautschichten kann auf geeignete Weise begrenzt werden, ohne dass es zu Verzögerungen bei der Behandlung kommt.

Figur 3 zeigt eine schematische Darstellung einer Anordnung bestehend aus einer Mehrzahl von bewegbaren Baugruppen jeweils mit Laserlichtquelle 2 und Optikeinheit 3. Die Optikeinheiten 3 verfügen, wie im Zusammenhang mit den Figuren 1 und 2 bereits erläutert, über einen Hohlspiegel 10 und einen konvexen Auffangspiegel 9, deren Spiegeloberflächen einander zugewandt sind und die eine Strahlführung, wie sie etwa aus Schwarzschild-Objektiven bekannt ist, ermöglichen. Die hierfür vorgesehene Steuereinheit 14 erzeugt auf der Grundlage der vom Anwender eingegebenen Daten und/oder von Parametern, die in einem Datenspeicher 15 abgelegt sind und Informationen über Haut, Gewebe und Strahleneigenschaften enthalten können, Steuersignale um die Laserlichtquellen 2, die Optikelemente 3 und/oder die Bewegungsmittel 13 auf geeignete Weise einstellen und einen Behandlungserfolg bei minimalen Schädigungen des gesunden Gewebes und der gesunden Haut sicher zu stellen. In diesem Zusammenhang wird mittels der zentralen Steuereinheit 14 auch der benötigte Abstand zwischen den Lichtaustrittsöffnungen 5 und der Hautoberfläche 8 eingestellt. Um eine geeignete Einstellung zu bewirken, übernimmt die Steuereinheit 14 zusammen mit entsprechend geeigneten Bewegungsmittel 13 zur gesteuerten Bewegung des Geräteträgers 21, der Laserlichtquellen 2 und/oder der Optikelement 3, insbesondere der Spiegel 9, 10 die Funktion des Stellmittels 7. Eine derartige automatisierte Einstellung unterschiedlicher Komponenten und damit des Abstandes zwischen den Lichtaustrittsöffnungen 5 und der Hautoberfläche 8 und damit auch zwischen der Hautoberfläche 8 und der Zielstruktur 1, um so einen Brennpunkt oder Brennfleck innerhalb der Zielstruktur 1 zu erzeugen, erfolgt bevorzugt mit Hilfe eines stationären Bestrahlungsgerätes.

Ebenso ist es möglich, eine derartige Anordnung zu miniaturisieren, so dass die Anordnung in einem handführbaren Gerät 18 Platz findet. Wesentlich ist jeweils, dass mit einer entsprechenden Anordnung, unabhängig von ihrer Baugröße entweder zeitgleich, zeitlich überlappend oder in zeitlichem Abstand zu einander wenigstens zwei Laserstrahlen 4 erzeugt werden, die wenigstens abschnittsweise auf unterschiedlichen Wegen oder zu unterschiedlichen Zeitpunkten den Körper des Patienten in Richtung der Zielstruktur 1 durchlaufen. Sofern eine entsprechende Anordnung in einem handführbaren Gerät verbaut wird, ist es von Vorteil, wenn dem Behandler, insbesondere über ein externes oder in das Handgerät integrierte Display, eine Information darüber ausgegeben wird, ob sich Gerät in der richtigen Position relativ zur Zielstruktur 1, insbesondere im richtigen Abstand zur Hautoberfläche 8 befindet. Die Information wird derart ausgegeben, dass es dem Benutzer auf einfache Weise möglich ist zu erkennen, ob und in welche Richtung das Gerät bewegt werden muss.

### Bezugszeichenliste

- 1: Zielstruktur
- 2: Laserlichtquelle
- 3: Optikelement
- 4: Laserlichtstrahlung
- 5: Lichtaustrittsöffnung
- 6: Brennpunkt / Brennfleck
- 7: Stellelement
- 8: Hautoberfläche
- 9: konvexer Spiegel
- 10: Hohlspiegel
- 11: Öffnung im Hohlspiegel
- 12: Abstandhalter
- 13: Bewegungsmittel
- 14: Steuereinheit
- 15: Datenspeicher
- 16: Gehäuse
- 17: Handgriff
- 18: Bestrahlungsgerät
- 19: Schaltelement
- 20: Diffusorelement
- 21: Geräteträger

## Patentansprüche

1. Vorrichtung zur Bestrahlung einer Zielstruktur (1), die sich innerhalb von menschlicher oder tierischer Haut oder Gewebe befindet, mit wenigstens einer Laserlichtquelle (2) und zumindest einem Optikelement (3), mittels dem die Laserstrahlung (4) durch eine Lichtaustrittsöffnung (5) geleitet und in einem Brennpunkt (6) außerhalb der Lichtaustrittsöffnung (5) fokussiert wird, wobei das Optikelement (3) eine Anordnung eines konvexen Spiegels (9) innerhalb eines Hohlspiegels (10) aufweist, die derart angeordnet sind, dass von der Laserlichtquelle (2) emittierte Laserstrahlung (4) zumindest teilweise durch eine Öffnung (11) im Hohlspiegel (10) hindurchtritt, die durch die Öffnung (10) hindurchgetretene Laserstrahlung (4) auf den konvexen Spiegel (9) trifft, vom konvexen Siegel (9) zum Hohlspiegel (10) gelenkt wird und vom Hohlspiegel (10) kommend die Lichtaustrittsöffnung (5) durchquert,
**dadurch gekennzeichnet, dass** wenigstens ein Stellelement (7) vorgesehen ist, mit dem der Abstand zwischen einer Hautoberfläche (8) und dem im Bereich der Zielstruktur (1) positionierten Brennpunkt (6) gezielt einstellbar ist, wobei eine Steuereinheit (14) zumindest zeitweise ein Steuersignal an das Stellelement (7) derart überträgt, dass eine Eindringtiefe der Laserstrahlung (4) in die Haut oder subkutanes Gewebe gezielt verändert wird,
wobei das Stellelement (7) als Abstandhalter (12) mit einem Aktuator ausgebildet ist und der Abstandhalter (12) wenigstens teilweise durch den Aktuator derart bewegt wird, dass wenigstens zwei unterschiedliche Abstände zwischen der Lichtaustrittsöffnung (5) und der Hautoberfläche einstellbar sind, und/oder
wobei das Stellelement (4) wenigstens ein Bewegungsmittel (13) aufweist, mit dem wenigstens ein Bauteil des Optikelements (3) derart bewegbar ist, dass der Abstand zwischen der Lichtaustrittsöffnung (5) und dem Brennpunkt (6) aufgrund der Bewegung verstellt wird.

2. Vorrichtung nach Anspruch 1,
**dadurch gekennzeichnet, dass** das Stellelement (7) über einen Abstandhalter (12) verfügt, der derart auf die Hautoberfläche (8) aufsetzbar ist, dass ein Abstand zwischen der Lichtaustrittsöffnung (5) und der Haut einstellbar ist.

3. Vorrichtung nach Anspruch 2,
**dadurch gekennzeichnet, dass** der Abstandhalter (12) einen Aktuator aufweist, über den der Abstandhalter (12) wenigstens teilweise derart bewegbar ist, dass wenigstens zwei unterschiedliche Abstände zwischen der Lichtaustrittsöffnung (5) und der Hautoberfläche (8) einstellbar sind.

4. Vorrichtung nach einem der Ansprüche 1 bis 3,
**dadurch gekennzeichnet, dass** das Stellelement (7) wenigstens ein Bewegungsmittel (13) aufweist, mit dem wenigstens ein Bauteil des Optikelements (3) derart bewegbar ist, dass der Abstand zwischen der Lichtaustrittsöffnung (5) und dem Brennpunkt (6) aufgrund der Bewegung verstellt wird.

5. Vorrichtung nach einem der Ansprüche 1 bis 4,
**dadurch gekennzeichnet, dass** zumindest ein Kühlelement vorgesehen ist, mit dem eine Hautoberfläche (8), insbesondere eine Oberfläche der Hornschicht, kühlbar ist.

6. Vorrichtung nach einem der Ansprüche 1 bis 5,
**dadurch gekennzeichnet, dass** eine Steuereinheit (14) vorgesehen ist, die zumindest zeitweise ein Steuersignal an die Laserlichtquelle (2), das Optikelement (3) und/oder das Stellelement (7) überträgt.

7. Vorrichtung nach Anspruch 6,
**dadurch gekennzeichnet, dass** in der Steuereinheit (14) oder einem an die Steuereinheit (14) gekoppelten Datenspeicher (15) Eigenschaften wenigstens einer Hautschicht und/oder eines Gewebetyps hinterlegt sind.

8. Vorrichtung nach Anspruch 6 oder 7,
**dadurch gekennzeichnet, dass** in der Steuereinheit (14) oder einem an die Steuereinheit (14) gekoppelten Datenspeicher (15) wenigstens eine optische Eigenschaft, insbesondere ein Wert für einen Brechungsindex und/oder für ein Absorptionsvermögen wenigstens eines Haut- und/oder Gewebebereichs hinterlegt ist.

9. Vorrichtung nach einem der Ansprüche 1 bis 8,
**dadurch gekennzeichnet, dass** die Laserlichtquelle (2) einen He-Ne-Laser, einen Nd-YAG-Laser und/oder einen Er-YAG-Laser aufweist.

10. Vorrichtung nach einem der Ansprüche 1 bis 9
**dadurch gekennzeichnet, dass** die Laserlichtquelle (2), das Optikelement (3) und
das Stellelement (7) von einem einteiligen oder mehrteiligen Gehäuse (16) umgeben sind, in dem sich die Lichtaustrittsöffnung (5) befindet und an dem ein Handgriff (17) zur manuellen Führung befestigt ist.

11. Vorrichtung nach einem der Ansprüche 1 bis 10,
**dadurch gekennzeichnet, dass** zwischen der Laserlichtquelle (2) und dem konvexen Spiegel (9) eine Diffusoroptik (20) angeordnet ist, durch die eine Intensitätsverteilung der Laserlichtstrahlung (4) derart verändert wird, dass die Laserlichtstrahlung (4) in einer Strahlmitte eine geringere Intensität als im Randbereich aufweist.

## Claims

1. Device for irradiating a target structure (1) located within human or animal skin or tissue, comprising at least one laser light source (2) and at least one optical element (3), by means of which the laser radiation (4) is guided through a light exit opening (5) and focused in a focal point (6) outside the light exit opening (5), wherein the optical element (3) has an arrangement of a convex mirror (9) within a concave mirror (10), which are arranged in such a way that laser radiation (4) emitted by the laser light source (2) passes at least partially through an opening (11) in the concave mirror (10), the laser radiation (4) passed through the opening (10) impinges on the convex mirror (9), is deflected from the convex mirror (9) to the concave mirror (10), and passes through the light exit opening (5) coming from the concave mirror (10), **characterized in that** at least one setting element (7) is provided with which the distance between a skin surface (8) and the focal point (6) positioned in the region of the target structure (1) can be set in a targeted manner, wherein a control unit (14) at least temporarily transmits a control signal to the setting element (7) in such a way that a penetration depth of the laser radiation (4) into the skin or subcutaneous tissue is changed in a targeted manner,
wherein the setting element (7) is designed as a spacer (12) with an actuator and the spacer (12) is at least partially moved by the actuator in such a way that at least two different distances between the light exit opening (5) and the skin surface can be set, and/or
wherein the setting element (7) has at least one movement means (13) with which at least one component of the optical element (3) can be moved in such a way that the distance between the light exit opening (5) and the focal point (6) is adjusted due to the movement.

2. Device according to Claim 1,
**characterized in that** the setting element (7) has a spacer (12) which can be placed on the skin surface (8) in such a way that a distance between the light exit opening (5) and the skin can be set.

3. Device according to Claim 2,
**characterized in that** the spacer (12) has an actuator via which the spacer (12) can be moved at least partially in such a way that at least two different distances between the light exit opening (5) and the skin surface (8) can be set.

4. Device according to any of Claims 1 to 3,
**characterized in that** the setting element (7) has at least one movement means (13) with which at least one component of the optical element (3) can be moved in such a way that the distance between the light exit opening (5) and the focal point (6) is adjusted as a result of the movement.

5. Device according to any of Claims 1 to 4,
**characterized in that** at least one cooling element is provided with which a skin surface (8), in particular a surface of the stratum corneum, can be cooled.

6. Device according to any of Claims 1 to 5,
**characterised in that** a control unit (14) is provided, which at least temporarily transmits a control signal to the laser light source (2), the optical element (3) and/or the setting element (7).

7. Device according to Claim 6,
**characterized in that** properties of at least one skin layer and/or tissue type are stored in the control unit (14) or in a data memory (15) coupled to the control unit (14).

8. Device according to Claim 6 or 7,
**characterized in that** at least one optical property, in particular a value for a refractive index and/or for an absorption capacity of at least one skin region and/or tissue region, is stored in the control unit (14) or in a data memory (15) coupled to the control unit (14).

9. Device according to any of Claims 1 to 8,
**characterized in that** said laser light source (2) comprises a He-Ne laser, an Nd-YAG laser and/or an Er-YAG laser.

10. Device according to any of Claims 1 to 9,
**characterized in that** the laser light source (2), the optical element (3) and the setting element (7) are surrounded by a single-part or multi-part housing (16) in which the light exit opening (5) is located and to which is attached a handle (17) for manual guidance.

11. Device according to any of Claims 1 to 10,
**characterized in that** a diffuser optical system (20) is arranged between the laser light source (2) and the convex mirror (9), via which an intensity distribution of the laser light radiation (4) is changed in such a way that the laser light radiation (4) has a lower intensity in a beam centre than in the edge region.

## Revendications

1. Dispositif pour irradier une structure cible (1) qui se trouve à l'intérieur d'une peau ou d'un tissu humain ou animal, avec au moins une source de lumière laser (2) et au moins un élément optique (3), au moyen duquel le rayonnement laser (4) est guidé à travers une ouverture de sortie de lumière (5) et focalisé en un point focal (6) à l'extérieur de l'ouverture de sortie de lumière (5), l'élément optique (3) présentant un agencement d'un miroir convexe (9) à l'intérieur d'un miroir creux (10), qui sont disposés de telle sorte que le rayonnement laser (4) émis par la source de lumière laser (2) traverse au moins partiellement une ouverture (11) dans le miroir creux (10), le rayonnement laser (4) traversant l'ouverture (10) rencontre le miroir convexe (9), est guidé par le miroir convexe (9) vers le miroir creux (10) et traverse l'ouverture de sortie de lumière (5) en provenant du miroir creux (10),
**caractérisé en ce qu'**au moins un élément de réglage (7) est prévu, avec lequel la distance entre une surface de peau (8) et le point focal (6) positionné dans la zone de la structure cible (1) est réglable de manière ciblée, une unité de commande (14) transmettant au moins temporairement un signal de commande à l'élément de réglage (7) de telle sorte qu'une profondeur de pénétration du rayonnement laser (4) dans la peau ou le tissu sous-cutané soit modifiée de manière ciblée,
dans lequel l'élément de réglage (7) est conçu comme un espaceur (12) avec un actionneur et l'espaceur (12) est déplacé au moins partiellement par l'actionneur de telle sorte qu'au moins deux distances différentes entre l'ouverture de sortie de lumière (5) et la surface de la peau sont réglables, et/ou
dans lequel l'élément de réglage (7) présente au moins un moyen de déplacement (13) avec lequel au moins un composant de l'élément optique (3) est déplaçable de telle sorte que la distance entre l'ouverture de sortie de lumière (5) et le point focal (6) est réglée en raison du mouvement

2. Dispositif selon la revendication 1,
**caractérisé en ce que** l'élément de réglage (7) dispose d'un espaceur (12) qui est plaçable sur la surface de la peau (8) de telle sorte qu'une distance entre l'ouverture de sortie de lumière (5) et la peau est réglable.

3. Dispositif selon la revendication 2,
**caractérisé en ce que** l'espaceur (12) présente un actionneur par lequel l'espaceur (12) est déplaçable au moins partiellement de telle sorte qu'au moins deux distances différentes entre l'ouverture de sortie de lumière (5) et la surface de la peau (8) sont réglables.

4. Dispositif selon l'une des revendications 1 à 3,
**caractérisé en ce que** l'élément de réglage (7) présente au moins un moyen de déplacement (13) avec lequel au moins un composant de l'élément optique (3) est déplaçable de telle sorte que la distance entre l'ouverture de sortie de lumière (5) et le point focal (6) est réglée en raison du mouvement

5. Dispositif selon l'une des revendications 1 à 4,
**caractérisé en ce qu'**au moins un élément de refroidissement est prévu avec lequel une surface de peau (8), en particulier une surface de la couche cornée, est refroidissable.

6. Dispositif selon l'une des revendications 1 à 5,
**caractérisé en ce que** une unité de commande (14) est prévue, qui transmet au moins temporairement un signal de commande à la source de lumière laser (2), à l'élément optique (3) et/ou à l'élément de réglage (7).

7. Dispositif selon la revendication 6,
**caractérisé en ce que** des propriétés d'au moins une couche de peau et/ou d'un type de tissu sont stockées dans l'unité de commande (14) ou dans une mémoire de données (15) couplée à l'unité de commande (14).

8. Dispositif selon la revendication 6 ou 7,
**caractérisé en ce qu'**au moins une propriété optique, en particulier une valeur pour un indice de réfraction et/ou pour une capacité d'absorption d'au moins une zone de la peau et/ou tissulaire, est stockée dans l'unité de commande (14) ou dans une mémoire de données (15) couplée à l'unité de commande (14).

9. Dispositif selon l'une des revendications 1 à 8,
**caractérisé en ce que** la source de lumière laser (2) comprend un laser He-Ne, un laser Nd-YAG et/ou un laser Er-YAG.

10. Dispositif selon l'une des revendications 1 à 9,
**caractérisé en ce que** la source de lumière laser (2), l'élément optique (3) et l'élément de réglage (7) sont entourés d'un boîtier (16) monobloc ou en plusieurs parties, dans lequel se trouve l'ouverture de sortie de lumière (5) et sur lequel est fixée une poignée (17) pour le guidage manuel.

11. Dispositif selon l'une des revendications 1 à 10,
**caractérisé en ce qu'**une optique de diffusion (20) est disposée entre la source de lumière laser (2) et le miroir convexe (9), par laquelle une répartition d'intensité du rayonnement de lumière laser (4) est modifiée de telle sorte que le rayonnement de lumière laser (4) présente une intensité plus faible dans un centre du faisceau que dans la zone périphérique.
